# EUROPEAN PATENT SPECIFICATION

(11) **EP 4 431 961 B1**
(45) Date of publication and mention of the grant of the patent: **25.03.2026**
(21) Application number: 24183642.8
(22) Date of filing: 18.02.2021
(51) Int. Cl.: A61B 1/00, G06F 3/048, H02J 7/00, B60L 58/10, G01R 31/367, G01R 31/382

(54) **BATTERY MONITORING FOR A MEDICAL VISUALISATION SYSTEM**
BATTERIEÜBERWACHUNG FÜR EIN MEDIZINISCHES VISUALISIERUNGSSYSTEM
SURVEILLANCE DE BATTERIE POUR UN SYSTÈME DE VISUALISATION MÉDICAL

(30) Priority: 21.02.2020 DK PA202070110
(43) Date of publication of application: 18.09.2024
(62) Divisional of application: 21707192.7
(73) Proprietor: Ambu A/S, 2750 Ballerup (DK)
(72) Inventor: UBBESEN, Line Sandahl, 2840 Holte (DK)
(74) Representative: COPA Copenhagen Patents

(56) References cited:
- US-A1- 2014 292 524
- US-A1- 2017 139 012
- US-B1- 6 494 827

## Description

The present disclosure relates to a visualisation device, such as an endoscope and a medical visualisation system, such as an endoscope system, comprising a visualisation device. More specifically the present disclosure relates to a graphical user interface and a monitor device having such graphical user interface for interacting with the medical visualisation system.

### BACKGROUND

A visualisation device may be utilized to visually examine certain areas of the body of a person, such as inside a body cavity of the person. For example, a visualisation device may be used to inspect the airways, the digestive tract, or the intestines.

A visualisation device may be provided with a camera and be attached to a monitor device, such as a monitor with a display screen, a video output from the camera of the visualisation device may be received and displayed at the monitor device, thereby allowing an operator to control the visualisation device to inspect an area of interest.

For example, a visualisation device may be an endoscope, such as a disposable endoscope. An endoscope comprises an operating handle at the proximal end and an insertion tube extending from the handle towards the distal end. The handle is configured to be held by an operator and inter alia comprises externally protruding operating members connected to internal control means allowing the operator to control the movement of a bending section at the distal end of the insertion tube, while advancing the distal end of the insertion tube to a desired location e.g. within a body cavity of a person. By means of an attached monitor device, such as a monitor with a display screen, the location to which the distal end has been advanced may be inspected using the endoscope.

The monitor device of a medical visualisation system may be provided with some functionality, such as ability to save still images and/or video sequences of the view from the attached visualisation device. Furthermore, the monitor device may comprise some image processing capabilities, and may be configured to output a video or image output, e.g. to an external display. Such a monitor device is disclosed in US 6494827 B1 and comprises a display and power supply for a visualisation device with an indication of the expected remaining battery time.

### SUMMARY

The present disclosure relates to a visualisation device, such as an endoscope, and a visualisation system, such as an endoscope system. Particularly, but not exclusively the visualisation device may be a disposable camera endoscope. Alternatively, the visualisation device may be a video laryngoscope, an endotracheal tube and/or a laryngeal mask. The visualisation system may further comprise a monitor device for being connected to the visualisation device, e.g. the monitor device may be configured to receive image data from the visualisation device. The present disclosure further relates to a graphical user interface for such monitor device of a medical visualisation system.

It is an object of the present disclosure to provide a solution which at least improve the solutions of the prior art. Particularly, it is an object of the present disclosure to provide a graphical user interface for a medical visualisation system which facilitates and enhances human interaction with the system.

It is a further object of the present disclosure to provide a system and method facilitating enhanced control and usability of a medical visualisation system.

Accordingly, a medical visualisation system and a method performed at a monitor device of the medical visualisation system are disclosed.

The medical visualisation system comprises a visualisation device, such as an endoscope, such as a disposable endoscope. Alternatively, the visualisation device may be a video laryngoscope, an endotracheal tube and/or a laryngeal mask. The visualisation device has an image sensor configured to generate image data indicative of a view from the visualisation device. The medical visualisation system may comprise a plurality of visualisation devices each having an image sensor configured to generate image data indicative of a view from the visualisation device. The plurality of visualisation devices may include a first visualisation device and/or a second visualisation device. The first visualisation device may comprise a first image sensor configured to generate image data indicative of a view from the first visualisation device. The second visualisation device may comprise a second image sensor configured to generate image data indicative of a view from the second visualisation device. The image sensor(s) may be any sensor capable of detecting and conveying information used to make an image. For example, the image sensor(s) may comprise a CCD or CMOS sensor, or similar. The image sensor(s) may generate image data corresponding to a square image, i.e. having equal height and width. For example, the image data generated by the image sensors may correspond to a 300x300 pixel image, or a 400x400 pixel image, or a 600x600 pixel image, or a 800x800 pixel image. Alternatively or additionally, the image sensor may generate image data corresponding to a non-square image, which is cropped to form a square image, such as a square image having 300x300 pixels, 400x400 pixels, 600x600 pixels, or 800x800 pixels.

The medical visualisation system further comprises a monitor device receiving and/or being operable to receive the image data generated by the image sensor. The monitor device may receive and/or be operable to receive the image data, e.g. as the image data is being generated, e.g. within limitation of the hardware. The monitor device comprises a first housing extending in a first direction from a first housing side to a second housing side and in a second direction perpendicular to the first direction from a third housing side to a fourth housing side. The monitor device comprises a display, e.g. a touch sensitive display. The display may be accommodated in the first housing. The display may have a first length in the first direction and a second length in the second direction. The second length may be longer than the first length, e.g. the display may be a 16:9 or 16:10 display. Alternatively, the first length may be longer than the second length, or the first length and the second length may be substantially the same. The touch sensitive display may be any suitable type of touch display, e.g. capacitive touch display or resistive touch display.

A monitor device according to the present invention is defined in the independent claim 1.

The monitor device may comprise one or more connection ports configured to receive a connector of the visualisation device. The connection ports and the corresponding connector of the visualisation device may be a proprietary plug and socket connectors, or any standard connector capable of transmitting therethrough at least the image data from the image sensor. Furthermore, the connector and connection ports may be configured to supply power to the components of the visualisation device.

The one or more connection ports may be arranged on the first housing. The one or more connection ports may be provided on the third housing side, and/or on the fourth housing side. The monitor device may comprise an on/off button. The on/off button may be arranged on the first housing. The on/off button may be provided on the third housing side or on the fourth housing side. The one or more connection ports may be provided on the third housing side and the on/off button may be provided on the fourth housing side.

The monitor device may establish connection to a visualisation device, such as the first visualisation device and/or the second visualisation device. Establishing connection to the visualisation device may include receiving a device connector of the respective visualisation device in a connection port of the one or more connection ports of the monitor device. Establishing connection to a visualisation device may include obtaining device identifier information from a device identifier (e.g. EPROM, QR-code, NFC, RFID or similar) of the visualisation device. For example, establishing connection to the first visualisation device may include obtaining first device identifier information from a first device identifier of the first visualisation device and/or establishing connection to the second visualisation device may include obtaining second device identifier information from a second device identifier of the second visualisation device.

The monitor device may comprise a processing unit and memory. The processing unit and/or the memory may be accommodated in the first housing. Alternatively, the monitor device may comprise a second housing, and the processing unit and/or the memory may be accommodated in the second housing. The monitor device may comprise an orientation sensor, e.g. for determining the orientation of the monitor device, such as of the first housing, relative to gravity. The orientation sensor may comprise one or more accelerometers and/or a gyroscope. The orientation sensor may be accommodated in the first housing. The processing unit may be connected to the touch sensitive display to control display of information with the touch sensitive display, and the processing unit may be adapted to receive a signal from the touch sensitive display indicative of touch inputs on the touch sensitive display. Thereby, the monitor device may detect user inputs, e.g. in the form of touch inputs, with the touch sensitive display. Touch inputs may, for example, comprise single tap(s), double tap(s), or swipe(s) on the touch sensitive display. The processing unit may be connected to the orientation sensor to receive an orientation signal indicative of the orientation of the monitor device, such as of the first housing of the monitor device. The processing unit may be connected to the memory and be adapted to read and write data from and to the memory.

The monitor device may comprise a power unit for powering the monitor device. The power unit may comprise a rechargeable battery and/or a power connection for connecting the power unit to an external power supply, such as a conventional AC power socket. The power unit may be accommodated in the first housing. Alternatively, the power unit may be accommodated in the second housing.

The monitor device may comprise a graphical user interface. The monitor device and/or the processing unit of the monitor device may display with the touch sensitive display the graphical user interface. The graphical user interface may comprise one or more portions, such as a plurality of portions. The portions may be non-overlapping portions, such as a plurality of non-overlapping portions. The portions may include a first portion and/or a second portion. The portions may further include a third portion and/or a fourth portion. The second portion and/or the fourth portion may be designated as background portions, e.g. the second portion may be a first background portion and/or the fourth portion may be a second background portion. Each of the plurality of portions may extend substantially throughout the first length in the first direction. The first portion may be arranged between the fourth portion and the second portion along the second direction. The fourth portion may be arranged between the third portion and the first portion along the second direction. The third portion may be arranged between a side of the first housing, e.g. the third housing side, and the fourth portion along the second direction. The second portion may be arranged between another side of the first housing, e.g. the fourth housing side, and the first portion along the second direction. The first portion and the fourth portion may be arranged between the second portion and the third portion along the second direction. The first portion of the graphical user interface may be square. The first portion of the graphical user interface may occupy the centre of the touch sensitive display. The first portion of the graphical user interface may be larger along the second direction than the second portion, the third portion and/or the fourth portion, individually and/or collectively. The first portion of the graphical user interface may extend throughout more than 40% of the second length in the second direction, such as more than 50% of the second length in the second direction, such as more than 60% of the second length in the second direction.

The monitor device may display a live representation of the image data, e.g. within the first portion of the graphical user interface. The live representation of the image data may be displayed, e.g. by the processing unit, with the touch sensitive display, e.g. within the first portion of the graphical user interface.

The visualisation device, e.g. an endoscope, may comprise a handle and an elongated flexible member extending from the handle to a distal end. The image sensor may be arranged at the distal end of the elongated flexible member. The image data may be indicative of a view from the distal end of the elongated flexible member. The handle may comprise a control button adapted to receive an input in a first input direction and/or in a second input direction. The first input direction and the second input direction may be opposite. The touch input in the first input direction may cause a distal portion of the elongated flexible member to bend in a first bending direction and/or may cause movement of the image sensor in a first image sensor direction. The touch input in the second input direction may cause the distal portion of the elongated flexible member to bend in a second bending direction and/or may cause movement of the image sensor in a second image sensor direction. The live representation of the image data may have directions corresponding to directions of the image sensor generating the image data. The first bending direction may correspond to a first image direction of a representation of the image data, such as the live representation of the image data. The second bending direction may correspond to a second image direction of the representation of the image data, such as the live representation of the image data. The first image direction and/or the second image direction may be parallel to the first direction of the first housing.

The monitor device may provide one or more actionable items. One or more actionable items may be displayed, e.g. by the processing unit, with the touch sensitive display, e.g. within the second portion of the graphical user interface. One or more actionable menu items may be displayed, e.g. by the processing unit, with the touch sensitive display, e.g. within the third portion of the graphical user interface. A battery indicator may be displayed, e.g. by the processing unit, with the touch sensitive display, e.g. within the third portion of the graphical user interface. A time indicator may be displayed, e.g. by the processing unit, with the touch sensitive display, e.g. within the third portion of the graphical user interface.

The one or more actionable items, e.g. displayed within the second portion of the graphical user interface of the monitor device, may comprise an image capture button and/or a video capture button. In response to activation of the image capture button, e.g. by a user providing a touch input, e.g. a single tap, at the respective location of the touch sensitive display, an image data file corresponding to the image data received when the image capture button was activated may be stored, e.g. in memory of the monitor device. In response to activation of the video capture button, e.g. by a user providing a touch input, e.g. a single tap, at the respective location of the touch sensitive display, a video sequence of image data corresponding to the image data received when the video capture button was activated may be stored, e.g. in memory of the monitor device. A first activation of the video capture button may start collection of image data for the video sequence, and a second activation of the video capture button (subsequent to the first activation of the video capture button) may stop the collection of image data for the video sequence. The stored video sequence may correspond to the image data received between the first activation and the second activation of the video capture button. The video capture button may be displayed in a first appearance prior to the first activation and after the second activation. The video capture button may be displayed in a second appearance after the first activation and before the second activation.

The monitor device may display a battery indicator, e.g. within the third portion of graphical user interface. The battery indicator may be indicative of a remaining charge, e.g. relative to full charge, of the rechargeable battery. The battery indicator may indicate an expected remaining battery time, e.g. the battery indicator may comprise a time indicator indicative of the expected remaining battery time, e.g. in hours and/or minutes. The expected remaining battery time may be the expected remaining battery time for which the monitor device is expected to be operational while being unplugged from the external power supply.

The expected remaining battery time may be provided based on whether a visualisation device is connected or not. For example, in accordance with the visualisation device not being connected to the monitor device, the expected remaining battery time may be based on expected power consumption with the visualisation device being connected; and in accordance with the visualisation device, and optionally a second visualisation device, being connected to the monitor device, the expected remaining battery time may be based on a measured power consumption, e.g. corresponding to power supplied by the rechargeable battery while the monitor device is unplugged from the external power supply. The measured power consumption may be an average of power consumption during a duration of time, e.g. 5 minutes, 10 minutes or 20 minutes.

The battery indicator may be displayed in one of a plurality of different states indicative of remaining charge of the rechargeable battery. In accordance with the expected remaining battery time being less than a low threshold amount of time and/or in accordance with the remaining charge being less than a low threshold charge, the battery indicator may be displayed in a low battery power state. In accordance with the expected remaining battery time being more than the low threshold amount of time and less than a high threshold amount of time and/or in accordance with the remaining charge being more than the low threshold charge and less than a high threshold charge, the battery indicator may be displayed in a medium battery power state. In accordance with the expected remaining battery time being more than the high threshold amount of time and/or in accordance with the remaining charge being more than the high threshold charge, the battery indicator may be displayed in a high battery power state. The low battery power state may comprise a red coloured part of the battery indicator. The medium battery power state may comprise a yellow-coloured part of the battery indicator. The high battery power state comprises a green coloured part of the battery indicator.

The high threshold charge may be between 30-50%, such as 40% of full charge. The high threshold charge may correspond to the high threshold amount of time. The high threshold amount of time may be between 1 hour to 1,5 hour, such as 1:12 or 1:15 (hours:minutes). The low threshold charge may be between 15-30%, such as 20% of full charge. The low threshold charge may correspond to the low threshold amount of time. The low threshold amount of time may be between 30-40 minutes, such as 36 minutes. The low threshold amount of time may be substantially equivalent to an expected time for a typical procedure using the medical visualisation system. Thereby, the operator before initiating a procedure is provided with valuable information of the battery status, advising whether it is wise to continue with the current monitor device on battery power.

The monitor device may display, e.g. with the display, an enlarged rendering of the battery indicator. The enlarged rendering of the battery indicator may be displayed in the fourth portion of the graphical user interface. The enlarged rendering of the battery indicator may be displayed concurrently with the battery indicator. The enlarged rendering of the battery indicator may be displayed in the fourth portion of the graphical user interface concurrently with the battery indicator being displayed within the third portion of the graphical user interface.

The enlarged rendering may be displayed in accordance with the expected remaining battery time being less than the low threshold amount of time and/or in accordance with the remaining charge being less than the low threshold charge. For example, the monitor device, e.g. the processing unit of the monitor device, may determine that the expected remaining battery time have dropped below the low threshold amount of time and/or that the remaining charge have dropped below the low threshold charge, and in response to the expected remaining battery time having dropped below the low threshold amount of time and/or that the remaining charge have dropped below the low threshold charge, the enlarged rendering may be displayed.

Alternatively or additionally, the enlarged rendering may be displayed in response to the monitor device being turned on. This may give the operator upon turning the monitor device on a quick notification of whether the monitor device can be expected to complete an upcoming procedure. After a first duration of time after the monitor device has been turned on, display of the enlarged rendering of the battery indicator may be ceased.

In accordance with the visualisation device being connected to the monitor device, the live representation of the image data may be displayed within the first portion of the graphical user interface. In accordance with the visualisation device not being connected to the monitor device, a guiding animation may be shown in the first portion of the graphical user interface instead of the live representation of the image data. The guiding animation may fill the first portion of the graphical user interface.

In accordance with the visualisation device not being connected to the monitor device, and in accordance with the expected remaining battery time being more than the low threshold amount of time and/or the remaining charge being more than the low threshold charge, an animation of connecting the visualisation device to the monitor device is displayed within the first portion of the graphical user interface, e.g. instead of the live representation of the image data.

In accordance with the visualisation device not being connected to the monitor device, and in accordance with the expected remaining battery time being less than the low threshold amount of time and/or the remaining charge being less than the low threshold charge, an animation of connecting the external power supply to the power connection is displayed within the first portion of the graphical user interface, e.g. instead of the animation of connecting the visualisation device to the monitor device. Thus, the operator is encouraged to connect the monitor device to an external power supply prior to initiating the procedure, e.g. prior to connecting the visualisation device.

In accordance with the expected remaining battery time being less than the low threshold amount of time and/or in accordance with the remaining charge being less than the low threshold charge, the on/off button of the monitor device may be flashing. Thereby providing a further notification to the operator that the monitor device cannot be expected to complete a procedure without being charged and/or connected to a power supply.

### BRIEF DESCRIPTION OF THE FIGURES

Embodiments of the disclosure will be described in more detail in the following with regard to the accompanying figures. The figures show one way of implementing the present invention and are not to be construed as being limiting to other possible embodiments falling within the scope of the attached claim set.
Fig. 1 schematically illustrates an exemplary medical visualisation system,
Fig. 2 schematically illustrates an exemplary monitor device,
Fig. 3 schematically illustrates an exemplary monitor device,
Fig. 4 is a block diagram of an exemplary monitor device,
Figs. 5A-5B schematically illustrates an exemplary graphical user interface of an exemplary monitor device,
Fig. 6 schematically illustrates exemplary battery indicators,
Figs. 7A-7B schematically illustrates an exemplary graphical user interface of an exemplary monitor device, and
Figs. 8A-8D schematically illustrates an exemplary graphical user interface of an exemplary monitor device.

### DETAILED DESCRIPTION

Various exemplary embodiments and details are described hereinafter, with reference to the figures when relevant. It should be noted that the figures may or may not be drawn to scale and that elements of similar structures or functions are represented by like reference numerals throughout the figures. It should also be noted that the figures are only intended to facilitate the description of the embodiments. They are not intended as an exhaustive description of the invention or as a limitation on the scope of the invention. In addition, an illustrated embodiment needs not have all the aspects or advantages shown. An aspect or an advantage described in conjunction with a particular embodiment is not necessarily limited to that embodiment and can be practiced in any other embodiments even if not so illustrated, or if not so explicitly described.

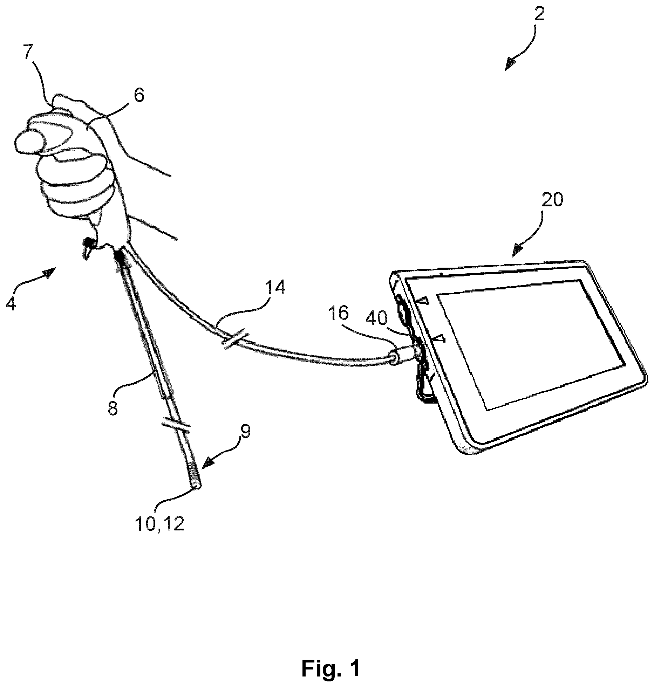

Fig. 1 schematically illustrates an exemplary medical visualisation system 2 comprising a visualisation device 4 and a monitor device 20. The visualisation device 4 has an image sensor 12, e.g. a CCD or a CMOS, configured to generate image data indicative of a view from the visualisation device 4. In the illustrated example, the visualisation device 4 is an endoscope comprising a handle 6 and an elongated flexible member 8, e.g. an insertion tube, extending from the handle 6 to a distal end 10. The image sensor 12 may be configured to generate image data indicative of a view from the distal end 10 of the elongated flexible member 8.

The visualisation device 4 may be connected to the monitor device 20. In the illustrated example, a device cable 14 extending from the handle 6 terminates in a device connector 16 connected to a connection port 40 of the monitor device 20. The monitor device 20 is operable to receive image data generated by the image sensor 12 of the visualisation device 4. For example, the monitor device 20 may receive image data generated by the image sensor 12 via the device cable 14, the connector 16 and connection port 40.

The handle 6 comprises a control button 7 adapted to receive an input in a first input direction and/or in a second input direction. The touch input in the first input direction on the control button 7 causes a distal portion 9 of the elongated flexible member 8 to bend in a first bending direction, e.g. via wires extending from the handle, through the elongated flexible member 8 to the distal portion 9. The touch input in the second input direction on the control button 7 causes the distal portion 9 of the elongated flexible member 8 to bend in a second bending direction. The first input direction and the second input direction may be opposite. The first bending direction and the second bending direction may be opposite. Bending the distal portion 9 of the elongated flexible member 8 may cause a movement of the distal end 10 and the image sensor 12 in a direction relative to the image sensor 12. Thereby, seeing an image generated by the image sensor 12, a direction, e.g. up or down, in the image may correspond to a respective input on the control button 7.

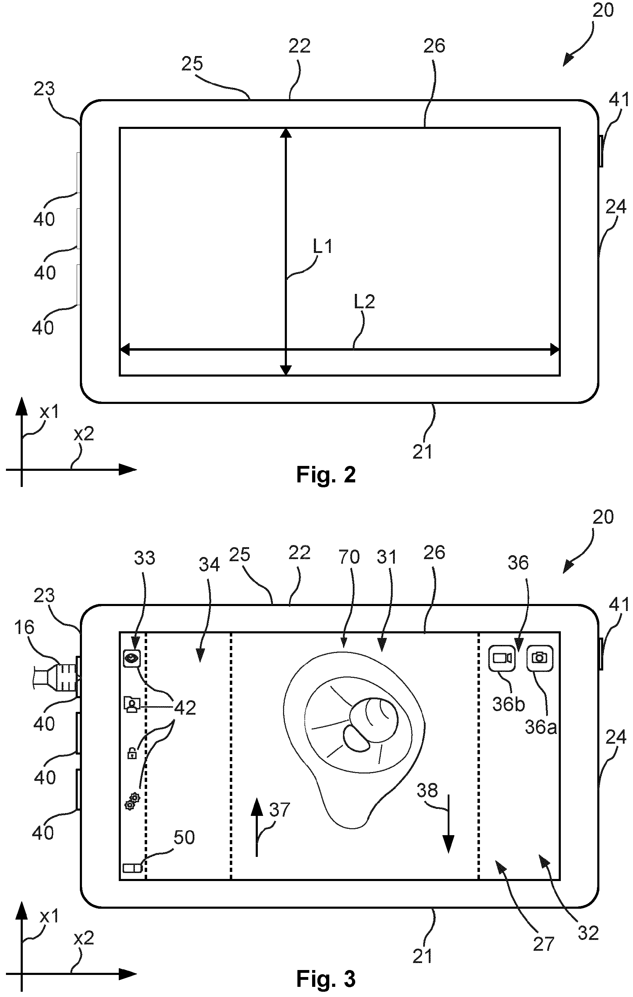

Fig. 2 schematically illustrates an exemplary monitor device 20, such as the monitor device 20 as illustrated in Fig. 1. The monitor device 20 comprises a first housing 25. The first housing 25 extends in a first direction x1 from a first housing side 21 to a second housing side 22 and in a second direction x2 perpendicular to the first direction x1 from a third housing side 23 to a fourth housing side 24. The monitor device comprises a touch sensitive display 26 accommodated in the first housing 25. The touch sensitive display 26 has a first length L1 in the first direction x1 and a second length L2 in the second direction x2. The second length L2 may be longer than the first length L1 as illustrated.

The monitor device may comprise one or more connection port(s) 40, such as three connection ports 40, as illustrated. The connection ports 40 may allow visualisation devices to be connected. The connection port(s) 40 may be arranged at the third housing side 23, as illustrated. Alternatively or additionally, connection port(s) 40 may be arranged at the fourth housing side 24.

The monitor device may comprise an on/off button 41, which may be provided on the fourth housing side 24, as illustrated.

Fig. 3 schematically illustrates an exemplary monitor device 20, such as the monitor device 20 as illustrated in Figs. 1-2. As illustrated a device connector 16 may be connected to a connection port 40.

The monitor device 20 may be provided with a graphical user interface 27. The graphical user interface 27 may be displayed with the touch sensitive display 26, and the user may interact with the graphical user interface 27, e.g. by means of providing touch inputs on the touch sensitive display 26.

The graphical user interface 27 is displayed with the touch sensitive display 26. The graphical user interface 27 comprises a plurality of non-overlapping portions 31, 32, 33, 34. Each of the portions 31, 32, 33, 34 extends substantially throughout the first length L1 in the first direction x1. The non-overlapping portions includes a first portion 31, a second portion 32, a third portion 33 and a fourth portion 34. The first portion 31 is arranged between the fourth portion 34 and the second portion 32 along the second direction x2. The fourth portion 34 is arranged between the third portion 33 and the first portion 31 along the second direction x2. The third portion 33 is arranged between a side of the first housing, e.g. the third housing side 23, and the fourth portion 34 along the second direction x2. The second portion 32 is arranged between another side of the first housing 25, e.g. the fourth housing side 24, and the first portion 31 along the second direction x2. The first portion 31 and the fourth portion 34 are arranged between the second portion 32 and the third portion 33 along the second direction.

The monitor device 20 displays a live representation 70 of the image data within the first portion 31 of the touch sensitive display 26. The first bending direction and the second bending direction of the distal portion 9 of the elongated flexible member 8, as described with respect to Fig. 1, may corresponds to a first image direction 37 and a second image direction 38 of the live representation 70, respectively. The first image direction 37 and the second image direction 38 may be parallel to the first direction x1, as illustrated. The first image direction 37 and the second image direction 38 may be opposite, as illustrated. Thereby, a user operating the control button 7 of visualisation device 2 may cause movement of the distal portion 9 of the elongated flexible member 8 to bend in a direction corresponding to the first image direction 37 or the second image direction 38 of the live representation 70.

The monitor device 20 displays with the touch sensitive display 26 one or more actionable items 36 within the second portion 32 of the graphical user interface 27. The actionable items 36 may comprise an image capture button 36a, e.g. for storing an image data file corresponding to the image data received when the image capture button 36a was activated. Alternatively or additionally, the actionable items 36 may comprise a video capture button 36b, e.g. for storing a video sequence of image data corresponding to the image data received when the video capture button 36b was activated.

The monitor device 20 displays with the touch sensitive display 26 one or more actionable menu items 42 within the third portion 33 of the graphical user interface 27. The actionable menu items 42 may, for example, comprise a login menu item for initiating a login procedure, a settings menu item for accessing a settings menu, an archive menu item for browsing an archive, and a default menu item for returning to a default view. Also a battery indicator 50 is displayed in the third portion 33.

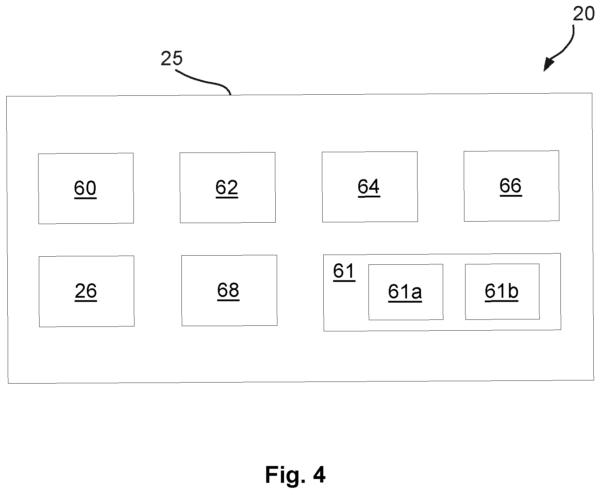

Fig. 4 is a block diagram of an exemplary monitor device 20, such as the monitor device 20 of the previous figures. The monitor device 20 comprises a processing unit 60 and memory 62. The memory 62 may comprise both volatile and non-volatile memory. The monitor device 20 also comprise an orientation sensor 64 for determining the orientation of the first housing 25 relative to gravity. The orientation sensor 64 may comprise one or more accelerometers and/or a gyroscope.

The monitor device 20 comprises input/output module 66, such as for receiving image data from the image sensor 12 via connectors of visualisation device 4. The input/output module 66 may also comprise ethernet connector, WiFi transceiver, Bluetooth transceiver, video connectors, USB ports etc., and respective controllers. The monitor device 20 also comprises the touch sensitive display 26 as described earlier. The monitor device 20 may display information, graphical user interface objects, images, buttons etc, with the touch sensitive display 27. The monitor device 20 also comprises a microphone 68. The monitor device 20 comprises a power unit 61 for powering the monitor device 20. The power unit 61 may comprise a rechargeable battery 61a. The power unit 61 may comprise a power connection 61b for connecting the power unit 61 to an external power supply, such as a conventional AC power socket. The components of the monitor device 20 may be interconnected by buses or signal lines. Some or all of the components of the monitor device may be accommodated in the first housing 25 as illustrated. However, alternatively some of the components, e.g. the processing unit 60, the memory 62, input/output module 66 and/or the power unit 61 may be accommodated in a second housing of the monitor device 20.

The power unit 61 may comprise components for, e.g. indirectly measuring, capacity of the rechargeable battery 61a. For example, the power unit 61 may comprise a voltage gauge to measure the voltage of the rechargeable battery 61a. Based on the measured voltage, the remaining capacity of the rechargeable battery 61a may be estimated, e.g. by the processing unit 60. The power unit 61 may also comprise components for measuring power consumption of the monitor device 20. For example, the power unit 61 may comprise a power meter to measure the rate at which the monitor device 20 consumes power from the rechargeable battery 61a. The voltage gauge may be a low current consumption integrated circuit or a resistor coupled in parallel with the battery. A current sensor may be provided, and the power may be computed as the product of the voltage and current. Additionally, an integrated circuit may be provided that includes a voltage gauge and a current sensor, and which outputs a power value in digital form.

The monitor device 20 may display content with the touch sensitive display 26. For example, the monitor device 20 may display content by the processing device 60 transmitting instructions to the touch sensitive display 26 indicative of the content to be displayed. The monitor device 20 may receive user input with the touch sensitive display 26. Particularly, the monitor device 20 may detect user inputs with the touch sensitive display 26. For example, a user providing a touch input on the touch sensitive display 26 causes a change in one or more electrical parameters of the touch sensitive display 26 indicative of at least the location of the touch input. Information of the touch input is transmitted from the touch sensitive display 26 to the processing unit 60, and the processing unit 60 may determine whether the touch input corresponds to an action to perform, e.g. whether the location of the touch input corresponds to the location of a soft-button displayed at the touch sensitive display.

The user may interact with the monitor device 20 via the graphical user interface 27 by providing user inputs, e.g. by means of providing touch inputs on the touch sensitive display 26, and the monitor device 20 may detect such user inputs with the touch sensitive display 26. A touch input, e.g. a single tap, double tap, swipe or similar, and the location of the touch input on the touch sensitive display 26 is registered by the touch sensitive display 26, which transmits information of the touch input (e.g. including type of touch (double tap, single tap, swipe, etc.) and/or location of the touch) to the processing unit 60 of the monitor device 20. The processing unit 60 interprets the information received and determines whether the touch input corresponds to activation of an action, e.g. whether the touch input correspond to activation of a button displayed with the touch sensitive display 27 at the location of the touch input. In response to a determination that the touch input corresponds to activation of an action, the processing unit 60 performs the respective action.

For example, with reference to Figs. 3 and 4, to capture an image corresponding to the presently shown live representation 70, e.g. corresponding to the image data received from the image sensor, the user may tap the image capture button 36a. The tap and the location of the tap is registered by the touch sensitive display 26, which transmits the information of the tap to the processing unit 60 of the monitor device 20. The processing unit 60 interprets the information received and determines that the user tapped the location corresponding to the image capture button 36a. In response thereto, the processing unit 60 stores, in memory 62 an image data file corresponding to the image data received.

In further reference to Figs. 3 and 4, to capture a video sequence corresponding to the shown live representation 70 over a period of time, e.g. corresponding to the image data received from the image sensor over a period of time, the user may tap the video capture button 36b. The tap and the location of the tap is registered by the touch sensitive display 26, which transmits the information of the tap to a processing unit 60 (see Fig. 4) of the monitor device 20. The processing unit 60 interprets the information received and determines that the user tapped the location corresponding to the video capture button 36b. In response thereto, the processing unit 60 starts collection of image data received from the image sensor 12 and temporarily stores the data in memory 62. To stop the recording, the user may tap the video capture button 36b again. The processing unit 60 determines, based on the signal received from the touch sensitive display 26, that that the user tapped the video capture button 36b and stops collecting image data received from the image sensor 12. The processing unit 60 read the temporarily stored data from the memory 62 and create a complete video sequence based thereon, which the processing unit 60 stores in the memory 62.

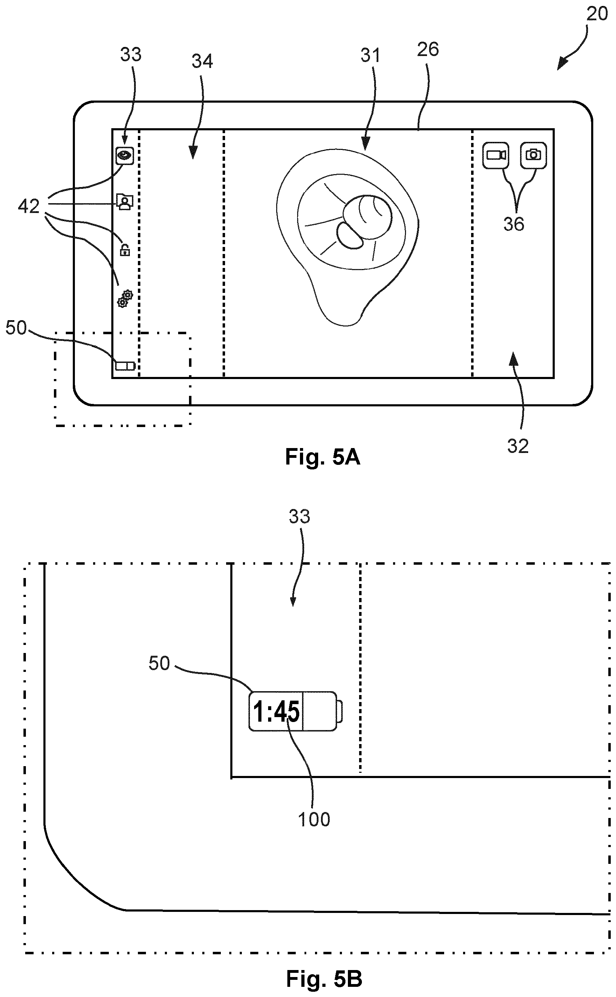

Fig. 5A illustrates an exemplary graphical user interface of a monitor device 20, such as the monitor device 20 of any of the previous figures. Fig 5B is an enlarged view of a part of the monitor device 20 of Fig. 5A, as illustrated by the dashed rectangle. As illustrated, the monitor device displays a battery indicator 50, e.g. in the third portion 33 of the touch sensitive display 26. The battery indicator 50 may be indicative of a remaining charge of the rechargeable battery 61a of the monitor device 20. For example, the battery indicator 50 comprises a time indicator 100 indicating an expected remaining battery time.

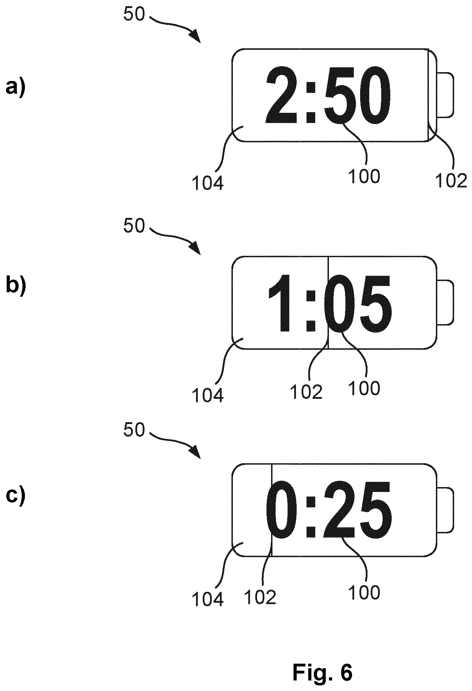

Fig. 6 schematically illustrates exemplary battery indicators 50. The battery indicator 50 may be displayed in one of a plurality of different states indicative of remaining charge of the rechargeable battery 61a of the monitor device 20. For example, the battery indicator 50 may be displayed in a high power state as exemplified in Fig. 6a, a medium power state as exemplified in Fig. 6b, and a low power state as exemplified in Fig. 6c. The battery indicator 50 comprises a time indicator 100 indicative of expected remaining battery time.

Remaining charge of the rechargeable battery may be estimated based on measuring of the battery voltage. As battery voltage drops capacity decreases. Knowing the characteristics of the rechargeable battery being provided in the monitor device 20, a certain voltage measurement may be used to estimate a certain remaining capacity of the rechargeable battery (e.g. measured in mAh, Ah, Wh or J), although factors, such as temperature, may influence the relationship between measured voltage and remaining capacity.

The expected remaining battery time may be estimated based on a consumption of power (e.g. measured in A or W). Dividing the remaining capacity (e.g. J) with the consumption of power (e.g. W) yields the expected remaining battery time.

The consumption of power used to calculate the expected remaining battery time, may be a measured consumption of power. For example, a component, e.g. of the power unit 61, may measure the present power consumption of the monitor device 20. The power unit 61 and/or the processing unit 60 may average the power consumption over a certain time frame, e.g. the previous 10 minutes. Alternatively or additionally, the consumption of power used to calculate the expected remaining battery time, may be a set value, or at least include a set value. For example, the consumption of power may be set to the power consumption the monitor device 20 is known to have in certain conditions. Such values may be found empirically. For example, the consumption of power used to calculate the expected remaining battery time may be set to an expected power consumption, e.g. corresponding to a consumption, e.g. empirically established, of the monitor device 20 with a single visualisation device being connected, e.g. wherein the monitor device is operating according to one or more established normal use cases, e.g. including functioning as a viewfinder for the visualisation device, powering the visualisation device, and/or capturing images and/or video sequences.

The expected remaining battery time, indicated by the battery indicator 50, may be calculated based on whether or not the visualisation device 4 is connected to the monitor device 10. For example, in accordance with the visualisation device not being connected to the monitor device, the expected remaining battery time may be based on expected power consumption with the visualisation device being connected, and in accordance with the visualisation device being connected to the monitor device, the expected remaining battery time may be based on a measured power consumption.

The high power state (Fig. 6a) may be displayed when the battery capacity is more than 40% and/or if the expected remaining battery time is more than a high threshold amount of time, e.g. between 1:00 (hours:mins) and 1:20, such as 1:12. The medium power state (Fig. 6b) may be displayed when the battery capacity is between 20-40% and/or if the expected remaining battery time is more than a low threshold amount of time and less than the high threshold amount of time. The low threshold amount of time may, e.g., be between 30 and 40 minutes, such as 36 minutes. The low power state (Fig. 6c) may be displayed when the battery capacity is less than 20% and/or less than the low threshold amount of time. The low threshold amount of time may be substantially equivalent to an expected time for a typical procedure using the medical visualisation system. Such expected time for a typical procedure may be empirically established. The inventors have found this to be about 36 minutes.

As illustrated in Fig. 6, when the expected remaining battery time decreases a battery level indicator 102 is moved to visually indicate the relative remaining battery capacity. Furthermore, the battery indicator 50, such as a part 104 of the battery indicator may change colour depending on the state of battery indicator. For example, in the high-power state as exemplified in Fig. 6a, the battery indicator 50 and/or the part 104 of the battery indicator 50 may be displayed in green. In the medium power state as exemplified in Fig. 6b, the battery indicator 50 and/or the part 104 of the battery indicator 50 may be displayed in yellow. In the low power state as exemplified in Fig. 6c, the battery indicator 50 and/or the part 104 of the battery indicator 50 may be displayed in red.

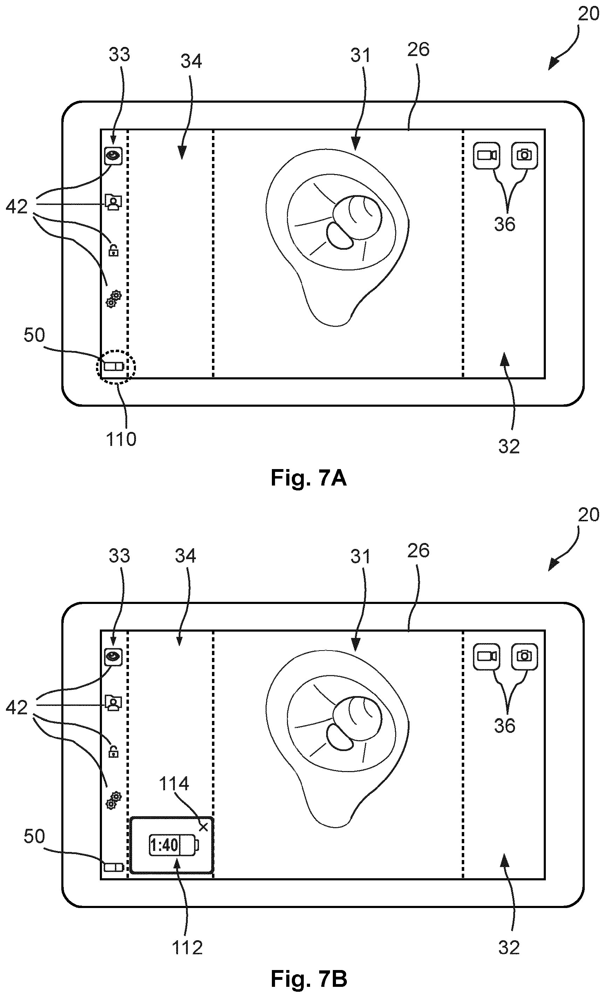

Figs. 7A-7B illustrates an exemplary graphical user interface of the monitor device 20, such as the same graphical user interface as described with respect to Fig. 5A. In Fig. 7A a user provides an input 110 to the battery indicator 50, e.g. the user provides a touch input 110 on the touch sensitive display 26 at the location corresponding to the battery indicator 50. The monitor device 20 detects the user input 110 with the touch sensitive display 26, and in response, as illustrated in Fig. 7B, an enlarged rendering 112 of the battery indicator 50 is displayed in the fourth portion 34 of the graphical user interface. Display of the enlarged rendering 112 may be ceased after a duration of time, e.g. 5 seconds after the monitor device detected the touch input 110. Alternatively, the user may manually close the enlarged rendering 112 by providing a touch input at the location of the cross 114 of the window containing the enlarged rendering 112.

The enlarged rendering 112 of the battery indicator 50 may also be displayed in accordance with the expected remaining battery time being less than the low threshold amount of time. For example, in response to the expected remaining battery time dropping below the low threshold amount of time, the enlarged rendering 112 of the battery indicator 50 may be displayed. The processing unit 60 may comprise instructions for updating the expected remaining battery time, and in response to a determination that the remaining battery time drops below the low threshold amount of time, the processing unit 60 may cause display of the enlarged rendering 112 of the battery indicator 50 on the touch sensitive display, e.g. within the fourth portion of the graphical user interface.

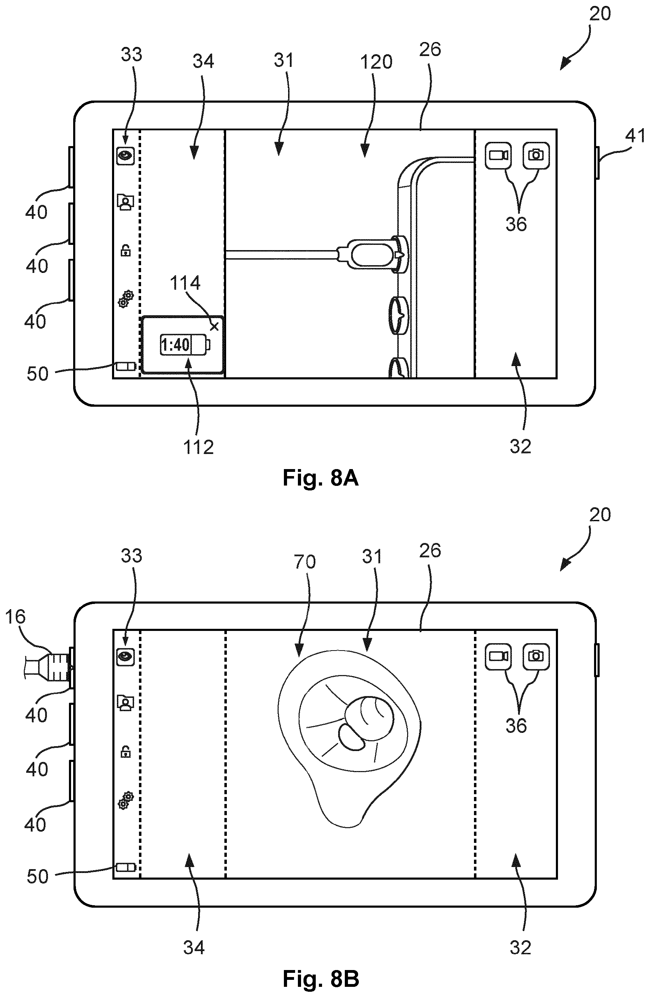

Figs. 8A-8D schematically illustrates an exemplary graphical user interface of a monitor device 20, such as the monitor device 20 of any of the previous figures. Particularly, Figs. 8A-8D illustrates that different content is displayed within the first portion 31 of the graphical user interface in accordance with whether a visualisation device is connected and depending on the expected remaining battery time.

Fig. 8A schematically illustrates an exemplary graphical user interface of a monitor device 20, wherein a visualisation device is not connected to the monitor device 20, and wherein the expected remaining battery time is more than the low threshold amount of time. For example, this situation may correspond to a situation where a user initially powers on the device, e.g. by pressing the on/off button 41. In this situation, based on that no visualisation device is connected and that the expected remaining battery time is more than the low threshold amount of time, an animation 120 of connecting the visualisation device to the monitor device 20 is displayed within the first portion 31 of the graphical user interface.

As also illustrated in Fig. 8A, an enlarged rendering 112 of the battery indicator 50 is displayed in the fourth portion 34 of the graphical user interface. The enlarged rendering 112 of the battery indicator 50 may be displayed in response to the monitor device 20 being turned on, notifying the user of the measured battery capacity, e.g. to let the user decide whether he/she wants to proceed with using the monitor device for the upcoming procedure. Display of the enlarged rendering 112 may be ceased after a duration of time, e.g. 5 seconds after the monitor device has been turned on. Alternatively, the user may manually close the enlarged rendering 112 by providing a touch input at the location of the cross 114 of the window containing the enlarged rendering 112.

Fig. 8B schematically illustrates an exemplary graphical user interface, e.g. following the graphical user interface as shown in Fig. 8A. In Fig. 8B, a visualisation device has been connected to the monitor device 20 by connecting the device connector 16 of the visualisation device to a connection port 40 of the monitor device 20. In this situation, because a visualisation device is connected, the live representation 70 of the image data is displayed within the first portion 31 of the graphical user interface.

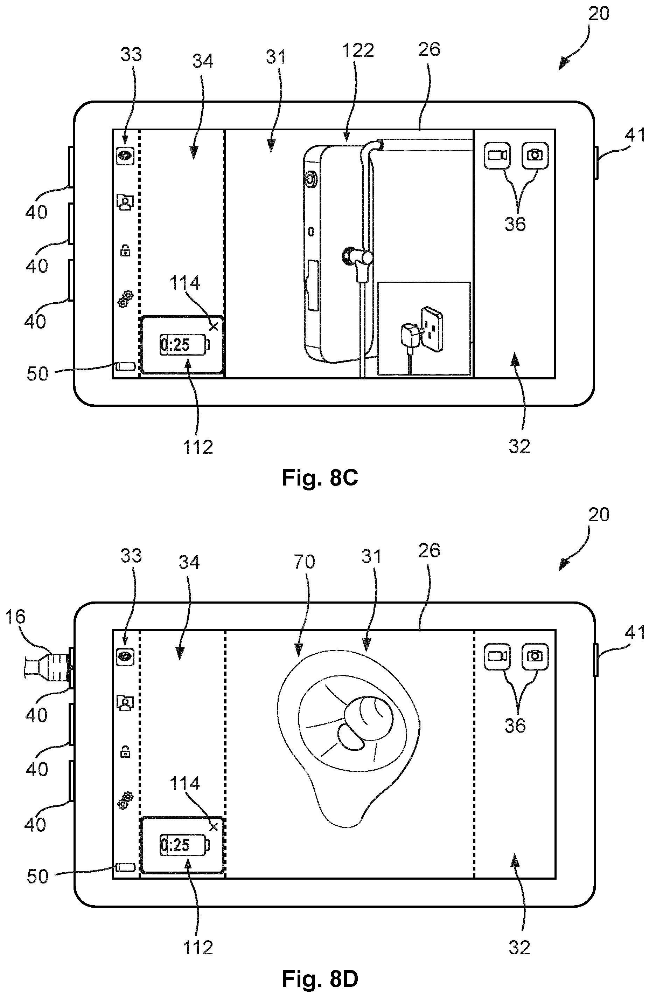

Fig. 8C schematically illustrates another example, similar to the example described with respect to Fig. 8A, wherein no visualisation device is connected to the monitor device 20. However, in Fig. 8C, the expected remaining battery time is less than the low threshold amount of time. In this situation, instead of displaying an animation 120 of connecting the visualisation device to the monitor device within the first portion 31 of the graphical user interface, as illustrated in Fig. 8A, an animation of connecting the external power supply to the power connection is displayed within the first portion of the graphical user interface. Furthermore, in accordance with the expected remaining battery time being less than the low threshold amount of time, the on/off button 41 of the monitor device 20 may be flashing.

Fig. 8D schematically illustrates an exemplary graphical user interface, e.g. following the graphical user interface as shown in Fig. 8C. In Fig. 8D, a visualisation device has been connected to the monitor device 20 by connecting the device connector 16 of the visualisation device to the connection port 40 of the monitor device 20. In this situation, because a visualisation device is connected, the live representation 70 of the image data is displayed within the first portion 31 of the graphical user interface, even though the expected remaining battery time is less than the low threshold amount of time. In this situation, the on/off button 41 may continue flashing. Optionally, the enlarged rendering 112 of the battery indicator 50 may be displayed until the user actively closes it by providing a touch input at the location of the cross 114 of the window containing the enlarged rendering 112.

The invention has been described with reference to preferred embodiments. However, the scope of the invention is not limited to the illustrated embodiments, and alterations and modifications can be carried out without deviating from the scope of the invention.

Throughout the description, the use of the terms "first", "second", "third", "fourth", "primary", "secondary", "tertiary" etc. does not imply any particular order of importance but are included to identify individual elements. Furthermore, the labelling of a first element does not imply the presence of a second element and vice versa.

### LIST OF REFERENCES

- 2: medical visualisation system
- 4: visualisation device
- 6: handle
- 7: control button
- 8: elongated flexible member
- 9: distal part
- 10: distal end of elongated flexible member
- 12: image sensor
- 14: device cable
- 16: device connector
- 20: monitor device
- 21: first housing side
- 22: second housing side
- 23: third housing side
- 24: fourth housing side
- 25: first housing
- 26: touch sensitive display
- 27: graphical user interface
- 31: first portion
- 32: second portion
- 33: third portion
- 34: fourth portion
- 36: actionable item(s)
- 37: first image direction
- 38: second image direction
- 40: connection port(s)
- 42: actionable menu item(s)
- 44: invert view button
- 46: inverted view mode indicator
- 50: battery indicator
- 60: processing unit
- 61: power supply
- 61a: battery
- 61b: power connection
- 62: memory
- 64: orientation sensor
- 66: input/output
- 68: microphone
- 70: live representation of image data
- x1: first direction
- x2: second direction
- L1: first length
- L2: second length

## Claims

1. A monitor device for a medical visualisation system comprising a visualisation device having an image sensor configured to generate image data indicative of a view from the visualisation device,
the monitor device being operable to receive the image data as the image data is being generated by the image sensor, the monitor device comprising a display, a graphical user interface and a power unit for powering the monitor device, the power unit comprising a rechargeable battery and a power connection for being connected to an external power supply, the monitor device displays the graphical user interface with the display,
wherein, in accordance with the visualisation device being connected to the monitor device, the monitor device displays a live representation of the image data within a first portion of the graphical user interface, and the monitor device displays a battery indicator indicative of a remaining charge of the rechargeable battery, wherein the battery indicator indicates an expected remaining battery time, wherein the battery indicator is displayed in one of a plurality of different states indicative of remaining charge of the rechargeable battery, and wherein, in accordance with the expected remaining battery time being less than a low threshold amount of time, the battery indicator is displayed in a low battery power state, and
wherein, in accordance with the visualisation device not being connected to the monitor device:
- in accordance with the expected remaining battery time being more than the low threshold amount of time, an animation of connecting the visualisation device to the monitor device is displayed within the first portion of the graphical user interface; and
- in accordance with the expected remaining battery time being less than the low threshold amount of time, an animation of connecting the external power supply to the power connection is displayed within the first portion of the graphical user interface.

2. Monitor device according to claim 1, wherein the low threshold amount of time is substantially equivalent to an expected time for a typical procedure using the medical visualisation system.

3. Monitor device according to any of the preceding claims, wherein, in accordance with the expected remaining battery time being less than the low threshold amount of time, the monitor device displays an enlarged rendering of the battery indicator.

4. Monitor device according to any of the preceding claims, wherein, in accordance with the expected remaining battery time being less than the low threshold amount of time, flashing an on/off button of the monitor device.

5. Monitor device according to any of the preceding claims, wherein the low battery power state comprises a red coloured part of the battery indicator.

6. Monitor device according to any of the preceding claims, wherein, in accordance with the expected remaining battery time being more than the low threshold amount of time and less than a high threshold amount of time, the battery indicator is displayed in a medium battery power state.

7. Monitor device according to claim 6, wherein the medium battery power state comprises a yellow-coloured part of the battery indicator.

8. Monitor device according to any of the preceding claims, wherein, in accordance with the expected remaining battery time being more than the high threshold amount of time, the battery indicator is displayed in a high battery power state.

9. Monitor device according to claim 8, wherein the high battery power state comprises a green coloured part of the battery indicator.

10. Monitor device according to any of the preceding claims, wherein:
- in accordance with the visualisation device not being connected to the monitor device, the expected remaining battery time is based on expected power consumption with the visualisation device being connected, and
- in accordance with the visualisation device being connected to the monitor device, the expected remaining battery time is based on a measured power consumption.

11. Monitor device according to claim 10, wherein in accordance with the visualisation device being connected to the monitor device and a second visualisation device being connected to the monitor device, the expected remaining battery time is based on the measured power consumption.

12. Monitor device according to any of the preceding claims, wherein the battery indicator is displayed within a third portion of the graphical user interface, the third portion and the first portion being non-overlapping.

13. Monitor device according to claim 12, wherein one or more actionable menu items is displayed within the third portion of the graphical user interface.

14. Monitor device according to any of the preceding claims, wherein, in response to the monitor device being turned on, the monitor device displays an enlarged rendering of the battery indicator, and, after a first duration of time after the monitor device has been turned on, the monitor device ceases display of the enlarged rendering of the battery indicator.

15. A medical visualisation system comprising a visualisation device having an image sensor configured to generate image data indicative of a view from the visualisation device, and a monitor device according to any of the preceding claims.

## Patentansprüche

1. Überwachungsvorrichtung für ein medizinisches Visualisierungssystem, umfassend eine Visualisierungsvorrichtung, die einen Bildsensor aufweist, der so konfiguriert ist, dass er Bilddaten erzeugt, die eine Sicht von der Visualisierungsvorrichtung angeben,
wobei die Überwachungsvorrichtung betriebsfähig ist, um die Bilddaten als die Bilddaten zu empfangen, die von dem Bildsensor erzeugt werden, wobei die Überwachungsvorrichtung eine Anzeige, eine grafische Benutzeroberfläche und ein Netzteil zum Versorgen der Überwachungsvorrichtung mit Strom umfasst, wobei das Netzteil eine wiederaufladbare Batterie und eine Stromverbindung zum Verbinden mit einer externen Stromversorgung umfasst, wobei die Überwachungsvorrichtung die grafische Benutzeroberfläche mit der Anzeige anzeigt,
wobei, wenn die Visualisierungsvorrichtung mit der Überwachungsvorrichtung verbunden ist, die Überwachungsvorrichtung eine Live-Darstellung der Bilddaten innerhalb eines ersten Abschnitts der grafischen Benutzeroberfläche anzeigt und die Überwachungsvorrichtung eine Batterieanzeige, die eine verbleibende Ladung der wiederaufladbaren Batterie angibt, anzeigt, wobei die Batterieanzeige eine erwartete verbleibende Batteriezeit angibt, wobei die Batterieanzeige in einem Zustand aus einer Vielzahl von unterschiedlichen Zuständen angezeigt wird, die verbleibende Ladung der wiederaufladbaren Batterie angeben, und wobei, wenn die erwartete verbleibende Batteriezeit weniger ist als eine niedrige Schwellenwert-Zeitmenge, die Batterieanzeige in einem Zustand niedrigen Batteriestroms angezeigt wird, und
wobei, wenn die Visualisierungsvorrichtung nicht mit der Überwachungsvorrichtung verbunden ist:
- wenn die erwartete verbleibende Batteriezeit mehr ist als die niedrige Schwellenwert-Zeitmenge, eine Animation des Verbindens der Visualisierungsvorrichtung mit der Überwachungsvorrichtung innerhalb des ersten Abschnitts der grafischen Benutzeroberfläche angezeigt wird; und
- wenn die erwartete verbleibende Batteriezeit weniger ist als die niedrige Schwellenwert-Zeitmenge, eine Animation des Verbindens der externen Stromversorgung mit der Stromverbindung innerhalb des ersten Abschnitts der grafischen Benutzeroberfläche angezeigt wird.

2. Überwachungsvorrichtung nach Anspruch 1, wobei die niedrige Schwellenwert-Zeitmenge im Wesentlichen gleichwertig zu einer erwarteten Zeit für ein typisches Verfahren unter Verwendung des medizinischen Visualisierungssystems ist.

3. Überwachungsvorrichtung nach einem der vorstehenden Ansprüche, wobei, wenn die erwartete verbleibende Batteriezeit weniger ist als die niedrige Schwellenwert-Zeitmenge, die Überwachungsvorrichtung eine vergrößerte Wiedergabe der Batterieanzeige anzeigt.

4. Überwachungsvorrichtung nach einem der vorstehenden Ansprüche, wobei, wenn die erwartete verbleibende Batteriezeit weniger ist als die niedrige Schwellenwert-Zeitmenge, einen Ein/Aus-Knopf auf der Überwachungsvorrichtung blinkt.

5. Überwachungsvorrichtung nach einem der vorstehenden Ansprüche, wobei der Zustand niedrigen Batteriestroms einen rot gefärbten Teil der Batterieanzeige umfasst.

6. Überwachungsvorrichtung nach einem der vorstehenden Ansprüche, wobei, wenn die erwartete verbleibende Batteriezeit mehr ist als die niedrige Schwellenwert-Zeitmenge und weniger als eine hohe Schwellenwert-Zeitmenge, die Batterieanzeige in einem mittleren Batteriestromzustand angezeigt wird.

7. Überwachungsvorrichtung nach Anspruch 6, wobei der mittlere Batteriestromzustand einen gelb gefärbten Teil der Batterieanzeige umfasst.

8. Überwachungsvorrichtung nach einem der vorstehenden Ansprüche, wobei, wenn die erwartete verbleibende Batteriezeit mehr ist als die hohe Schwellenwert-Zeitmenge, die Batterieanzeige in einem hohen Batteriestromzustand angezeigt wird.

9. Überwachungsvorrichtung nach Anspruch 8, wobei der hohe Batteriestromzustand einen grün gefärbten Teil der Batterieanzeige umfasst.

10. Überwachungsvorrichtung nach einem der vorstehenden Ansprüche, wobei:
- wenn die Visualisierungsvorrichtung nicht mit der Überwachungsvorrichtung verbunden ist, die erwartete verbleibende Batteriezeit auf einem erwarteten Stromverbrauch basiert, wenn die Visualisierungsvorrichtung verbunden wird, und
- wenn die Visualisierungsvorrichtung mit der Überwachungsvorrichtung verbunden ist, die erwartete verbleibende Batteriezeit auf einem gemessenen Stromverbrauch basiert.

11. Überwachungsvorrichtung nach Anspruch 10, wobei, wenn die Visualisierungsvorrichtung mit der Überwachungsvorrichtung verbunden ist und eine zweite Visualisierungsvorrichtung mit der Überwachungsvorrichtung verbunden ist, die erwartete verbleibende Batteriezeit auf dem gemessenen Stromverbrauch basiert.

12. Überwachungsvorrichtung nach einem der vorstehenden Ansprüche, wobei die Batterieanzeige innerhalb eines dritten Abschnitts der grafischen Benutzeroberfläche angezeigt wird, wobei der dritte Abschnitt und der erste Abschnitt nicht überlappend sind.

13. Überwachungsvorrichtung nach Anspruch 12, wobei ein oder mehrere betätigbare Menüelemente innerhalb des dritten Abschnitts der grafischen Benutzeroberfläche angezeigt werden.

14. Überwachungsvorrichtung nach einem der vorstehenden Ansprüche, wobei die Überwachungsvorrichtung als Reaktion darauf, dass die Überwachungsvorrichtung eingeschaltet wird, eine vergrößerte Wiedergabe der Batterieanzeige anzeigt, und die Überwachungsvorrichtung nach einer ersten Zeitspanne nach dem Einschalten der Überwachungsvorrichtung die Anzeige der vergrößerten Wiedergabe der Batterieanzeige beendet.

15. Medizinisches Visualisierungssystem, umfassend eine Visualisierungsvorrichtung, die einen Bildsensor aufweist, der so konfiguriert ist, dass er Bilddaten erzeugt, die eine Sicht von der Visualisierungsvorrichtung angeben, und eine Überwachungsvorrichtung nach einem der vorstehenden Ansprüche.

## Revendications

1. Dispositif de surveillance pour un système de visualisation médicale comprenant un dispositif de visualisation présentant un capteur d'image configuré pour générer des données d'image indiquant une vue à partir du dispositif de visualisation,
le dispositif de surveillance étant utilisable pour recevoir les données d'image pendant que les données d'image sont générées par le capteur d'image, le dispositif de surveillance comprenant une unité d'affichage, une interface utilisateur graphique et une unité d'alimentation pour alimenter le dispositif de surveillance, l'unité d'alimentation comprenant une batterie rechargeable et une connexion d'alimentation destinée à être connectée à une alimentation électrique externe, le dispositif de surveillance affiche l'interface utilisateur graphique avec l'unité d'affichage,
dans lequel, en fonction du dispositif de visualisation qui est connecté au dispositif de surveillance, le dispositif de surveillance affiche une représentation en direct des données d'image dans une première partie de l'interface utilisateur graphique, et le dispositif de surveillance affiche un indicateur de batterie indiquant une charge restante de la batterie rechargeable, dans lequel l'indicateur de batterie indique un temps de batterie restant attendu, dans lequel l'indicateur de batterie est affiché dans l'un parmi une pluralité d'états différents indiquant une charge restante de la batterie rechargeable, et dans lequel, en fonction du temps de batterie restant attendu qui est inférieur à une période de temps de seuil bas, l'indicateur de batterie est affiché dans un état de puissance de batterie faible, et dans lequel, en fonction du dispositif de visualisation qui n'est pas connecté au dispositif de surveillance :
- en fonction du temps de batterie restant attendu qui est supérieur à la période de temps de seuil bas, une animation de connexion du dispositif de visualisation au dispositif de surveillance est affichée à l'intérieur de la première partie de l'interface utilisateur graphique ; et
- en fonction du temps de batterie restant attendu qui est inférieur à la période de temps de seuil bas, une animation de connexion de l'alimentation électrique externe à la connexion d'alimentation est affichée à l'intérieur de la première partie de l'interface utilisateur graphique.

2. Dispositif de surveillance selon la revendication 1, dans lequel la période de temps de seuil bas est sensiblement équivalente à un temps attendu pour une procédure typique utilisant le système de visualisation médicale.

3. Dispositif de surveillance selon l'une quelconque des revendications précédentes, dans lequel, en fonction du temps de batterie restant attendu qui est inférieur à la période de temps de seuil bas, le dispositif de surveillance affiche un rendu agrandi de l'indicateur de batterie.

4. Dispositif de surveillance selon l'une quelconque des revendications précédentes, dans lequel, en fonction du temps de batterie restant attendu qui est inférieur à la période de temps de seuil bas, un bouton marche/arrêt du dispositif de surveillance clignote.

5. Dispositif de surveillance selon l'une quelconque des revendications précédentes, dans lequel l'état de puissance de batterie faible comprend une partie colorée rouge de l'indicateur de batterie.

6. Dispositif de surveillance selon l'une quelconque des revendications précédentes, dans lequel, en fonction du temps de batterie restant attendu qui est supérieur à la période de temps de seuil bas et inférieur à une période de temps de seuil haut, l'indicateur de batterie est affiché dans un état de puissance de batterie moyenne.

7. Dispositif de surveillance selon la revendication 6, dans lequel l'état de puissance de batterie moyenne comprend une partie de couleur jaune de l'indicateur de batterie.

8. Dispositif de surveillance selon l'une quelconque des revendications précédentes, dans lequel, en fonction du temps de batterie restant attendu qui est supérieur à la période de temps de seuil haut, l'indicateur de batterie est affiché dans un état de puissance de batterie élevée.

9. Dispositif de surveillance selon la revendication 8, dans lequel l'état de puissance de batterie élevée comprend une partie de couleur verte de l'indicateur de batterie.

10. Dispositif de surveillance selon l'une quelconque des revendications précédentes, dans lequel :
- en fonction du dispositif de visualisation qui n'est pas connecté au dispositif de surveillance, le temps de batterie restant attendu est basé sur la consommation d'énergie attendue avec le dispositif de visualisation qui est connecté, et
- en fonction du dispositif de visualisation qui est connecté au dispositif de surveillance, le temps de batterie restant attendu est basé sur une consommation d'énergie mesurée.

11. Dispositif de surveillance selon la revendication 10, dans lequel, en fonction du dispositif de visualisation qui est connecté au dispositif de surveillance et à un second dispositif de visualisation qui est connecté au dispositif de surveillance, le temps de batterie restant attendu est basé sur la consommation d'énergie mesurée.

12. Dispositif de surveillance selon l'une quelconque des revendications précédentes, dans lequel l'indicateur de batterie est affiché dans une troisième partie de l'interface d'utilisateur graphique, la troisième partie et la première partie ne se chevauchant pas.

13. Dispositif de surveillance selon la revendication 12, dans lequel un ou plusieurs éléments de menu actionnables sont affichés dans la troisième partie de l'interface d'utilisateur graphique.

14. Dispositif de surveillance selon l'une quelconque des revendications précédentes, dans lequel, en réponse au dispositif de surveillance qui est mis en marche, le dispositif de surveillance affiche un rendu agrandi de l'indicateur de batterie, et, après une première durée pendant laquelle le dispositif de surveillance a été mis en marche, le dispositif de surveillance cesse d'afficher le rendu agrandi de l'indicateur de batterie.

15. Système de visualisation médicale comprenant un dispositif de visualisation présentant un capteur d'image configuré pour générer des données d'image indiquant une vue à partir du dispositif de visualisation, et un dispositif de surveillance selon l'une quelconque des revendications précédentes.
